# EUROPEAN PATENT SPECIFICATION

(11) **EP 3 222 719 B1**
(45) Date of publication and mention of the grant of the patent: **22.04.2020**
(21) Application number: 16162349.1
(22) Date of filing: 24.03.2016
(51) Int. Cl.: C12N 15/10

(54) **USE OF AN AQUEOUS COMPOSITION FOR DISSOLVING BIOMOLECULES FROM A TISSUE SAMPLE**
VERWENDUNG EINER WÄSSRIGEN ZUSAMMENSETZUNG ZUM AUFLÖSEN VON BIOMOLEKÜLEN AUS EINER GEWEBEPROBE
UTILISATION D'UNE COMPOSITION AQUEUSE POUR LA DISSOLUTION DE BIOMOLÉCULES D'UN ÉCHANTILLON DE TISSU

(43) Date of publication of application: 27.09.2017
(73) Proprietor: Allflex Europe SA, 35500 Vitré (FR)
(72) Inventor: DE MEULEMEESTER, Johan, 35500 Vitré (FR); LEMAIRE, Emmanuel, 35500 Vitré (FR)
(74) Representative: Engelhard, Markus

(56) References cited:
- EP-A1- 1 088 212
- WO-A1-99/12475
- WO-A1-2016/029020
- WO-A2-2009/085355
- WO-A2-2012/054613
- Kay ET AL: "Standard Operating Procedure Tissue sample collection and storage for mammals", , 1 December 2015 (2015-12-01), XP055304780, Retrieved from the Internet: URL:https://www.dpaw.wa.gov.au/images/docu ments/plants-animals/monitoring/sop/sop08. 4_tissuesample_v1.0.pdf [retrieved on 2016-09-22]
- MICHAEL A. GRAY ET AL: "Comparison of DNA preservation methods for environmental bacterial community samples", FEMS MICROBIOLOGY ECOLOGY., vol. 83, no. 2, 8 October 2012 (2012-10-08), pages 468-477, XP055304779, NL ISSN: 0168-6496, DOI: 10.1111/1574-6941.12008

## Description

The present invention relates to the use of an aqueous composition for dissolving biomolecules from a tissue sample and to a method of using an aqueous composition for dissolving biomolecules from a tissue sample. Furthermore, the present invention relates to a system for preparing a tissue sample of an animal.

Tissue sampling tags are nowadays commonly used for tagging a livestock animal and at the same time withdrawing a sample from said animal. Such sample is then subsequently analyzed in the laboratory for the presence or absence of particular genetic traits, pathogens, or for genotyping said animal. Alternatively, the sample is simply kept and stored for tracing the origin of the animal from which said sample has been taken. Tissue samples may be obtained by using a tissue sampling tag or a tissue sampling unit, such as a biopsy needle. Tissue sampling tags are commonly known and are also commercially available, for example from the present applicant, Allflex Europe S.A..

In order to further analyze the sample, it is necessary to further process it. This is typically done in a laboratory to which the sample is transported after having been obtained from an animal. WO 99/12475 discloses a sample capsule of an ear tag which contains unspecified reagents for further processing of the sample. EP 1 088 212 discloses an ear tag which comprises a sample receiving container in which material for inactivating a protein part of a tissue sample is contained. As examples for such material for inactivating the protein part of a tissue sample the following are mentioned: proteinase K, a strongly alkaline solution, a molecular sieve and other components supporting an inactivation of the protein part in a sample. According to this approach, the tissue sample is contained in the tissue sample receiving container where its protein part is inactivated. The sample is transported to a laboratory where the actual analysis of the sample takes place. The procedure as disclosed in the prior art is laborious and time-consuming. It requires multiple work-up steps in the laboratory and eventually may lead to a total loss of the sample.

The present invention has been made to improve the process according to the prior art. Accordingly, it was an object of the present invention to provide for a methodology allowing a faster processing of the tissue samples. It was furthermore an object of the present invention to provide a methodology that allows to store the tissue sample for longer periods of time whilst, at the same time, being able to perform a swift analysis of the sample in terms of particular genetic trades, its genotype, the presence or absence of certain pathogens, such as BVDV.

All these objects are solved by the use of an aqueous composition for dissolving biomolecules selected from nucleic acids, preferably DNA, and proteins, from a tissue sample of an animal and for subsequent
a) preserving said biomolecules or
b) further processing said biomolecules,
wherein said use comprises the steps:
- sampling a tissue of an animal to produce a tissue sample of said animal and
- immediately after sampling, exposing said tissue sample to an aqueous composition by contacting said sample with said composition for a defined period of time, said composition comprising
- a buffer capable of buffering at a pH range from 7 to 9 at 25°C, optionally including a pH-setting agent, such as an acidifying agent or alkalizing agent,
- a detergent
- a salt at a concentration of 5-10 wt.%
- a chelating agent, and water,
wherein said tissue of an animal is sampled using a tissue sampling tag, preferably ear tag, or a tissue sampling biopsy needle, and wherein said sample is contacted with said composition by introducing said sample into said composition, and wherein by exposing said sample to said composition, biomolecules selected from nucleic acids and proteins, from said tissue sample become dissolved in said aqueous composition to produce a biomolecule solution,
- using said biomolecule solution for preserving said biomolecules or for further processing said biomolecules, such as detection of pathogen(s), e. g. BVDV, or genotyping, sequencing, hybridization analysis, or quantitative real-time PCR, or using said biomolecule solution for the detection of marker proteins, or marker nucleic acids, drugs, hormones or metabolites,
- optionally using part of said aqueous composition to store said tissue sample for later use, wherein said aqueous composition does not contain a proteinase.

In one embodiment, said aqueous composition also comprises an alkalizing agent, such as an alkali hydroxide, more preferably NaOH or KOH.

In one embodiment, said salt is NaCl which is present at a concentration of 1M to 2M, preferably 1M to 1.5M, more preferably 1.3M to 1.5M, even more preferably 1.4M.

In one embodiment, said detergent in said composition is N-lauroylsarcosine, preferably N-lauroylsarcosine sodium salt.

In one embodiment, said buffer is Tris and/or said chelating agent is EDTA, preferably EDTA disodium dihydrate.

In one embodiment, said aqueous composition comprises, preferably consists of the following components:

| | |
|---|---|
| Tris | 5-20 mM, preferably 10-15 mM |
| NaOH | 5-20 mM, preferably 8-10 mM |
| N-lauroylsarcosine | 2-15 mM, preferably 5-8 mM, more preferably 6-7 mM |
| sodium salt | |
| EDTA · 2 Na·2H₂O | 1-5 mM, preferably 1-3 mM |
| NaCl | 1-3 M, preferably 1-2 M, more preferably 1-1.5 M, more preferably 1.3-1.5 M, water, |

and, optionally, a dye indicating the presence of biomolecules, preferably nucleic acids.

In one embodiment, said aqueous composition comprises, preferably consists of the following components:

| | |
|---|---|
| Tris | 13 mM |
| NaOH | 8.5-8.6 mM, preferably 8.55 mM |
| EDTA · 2 Na·2H₂O | 1.9-2.1 mM, preferably 1.99-2 mM |
| N-lauroylsarcosine | 6-7 mM, preferably 6.7-6.9 mM, more preferably 6.8 mM |
| sodium salt | |
| NaCl | 1.35-1.45 M, preferably 1.38-1.42 M, and water |

In one embodiment, said biomolecule solution is used for further processing of said biomolecules, wherein said further processing is a detection of BVDV in said biomolecule solution and thus in said tissue sample, wherein, preferably, said detection of BVDV is done via nucleic acid amplification and detection of said amplified nucleic acids or via antibody-based detection of BVDV-proteins, such as ELISA of BVDV-proteins.

The objects of the present invention are also solved by a system for preparing a tissue sample of an animal for subsequent
a) genotyping said tissue,
b) detection of a pathogen in said tissue, or
c) storing and preserving said tissue sample for later use,
said system comprising a tissue sampling tag, preferably tissue sampling ear tag, or a tissue sampling biopsy needle, and an aqueous composition for dissolving biomolecules from a tissue sample of an animal, said composition being contained in container, said composition being as defined above.

The term "system for preparing a tissue sample of an animal", as used herein, is meant to refer to a combination of products which are operably linked with each other. The products that are included in such system are a tissue sampling tag, preferably a tissue sampling ear tag, or, instead of the tissue sampling tag, a tissue sampling biopsy needle, and, operably linked therewith, an aqueous composition for dissolving biomolecules from a tissue sample in accordance with the present invention. The term "operably linked", as used herein, may refer to a physical connection between the tissue sampling tag or tissue sampling biopsy needle, on the one hand, and the aqueous composition on the other hand, or it may refer to an arrangement, wherein the aqueous composition in accordance with the present invention is provided such that, after sampling of a tissue sample, such tissue sample may be immediately contacted with the aqueous composition in accordance with the present invention, without such composition being in physical contact with the tissue sampling tag or parts thereof. The term "to contact with", as used herein in the context of contacting the sample with the composition, may refer to an activity whereby the tissue sample is brought into physical connection with the aqueous composition. Such activity may be rinsing, soaking, imbibing, submersing, bathing, dunking, dipping or fully or partially covering the tissue sample with said aqueous composition. The term "tissue sampling tag", as used herein, refers to a device which is capable of providing an animal with a tag or mark (that remains with, in, on or attached in any other way to the animal) whilst concurrently therewith obtaining a tissue sample from the animal for subsequent use. A "tissue sampling unit" or, synonymously, a "tissue sampling biopsy needle", as used herein, is meant to refer to an instrument by which, for example through a scraping, punching, tearing or other action, a tissue sample can be obtained from an animal, without, however, attaching a tag or mark, such as a tag made from plastic, metal or wood, to said animal.

The objects of the present invention are also solved by a method of using an aqueous composition for dissolving biomolecules selected from nucleic acids, preferably DNA, and proteins, from a tissue sample of an animal and for subsequent
a) preserving said biomolecules or
b) further processing said biomolecules,
wherein said method comprises the steps:
- sampling a tissue of an animal to produce a tissue sample of said animal and
- immediately after sampling, exposing said tissue sample to an aqueous composition by contacting said sample with said composition for a defined period of time, said composition comprising
- a buffer capable of buffering at a pH range from 7 to 9 at 25°C, optionally including a pH-setting agent, such as an acidifying agent or an alkalizing agent.
- a detergent,
- a salt at a concentration of 5-10 wt.%,
- a chelating agent, and water,
- wherein said tissue of an animal is sampled using a tissue sampling tag, preferably ear tag, or a tissue sampling biopsy needle, and wherein said sample is contacted with said aqueous composition by introducing said sample into said composition, and wherein by exposing said sample to said aqueous composition, biomolecules selected from nucleic acids and proteins from said tissue sample become dissolved in said aqueous composition to produce a biomolecule solution,
- using said biomolecule solution for preserving said biomolecules or for further processing said biomolecules, such as detection of pathogen(s), e. g. BVDV, or genotyping, sequencing, hybridization analysis, or quantitative real-time PCR, or using said biomolecule solution for the detection of marker proteins, or marker nucleic acids, drugs, hormones or metabolites,
- optionally using said aqueous composition to store said tissue sample for later use, wherein said aqueous composition does not contain a proteinase.

The present inventors have surprisingly found that it is possible to initiate a processing of a tissue sample immediately after it has been obtained from an animal, by exposing it immediately to a suitable aqueous composition according to the present invention. In accordance with embodiments of the present invention, such suitable aqueous composition is characterized by high salt concentrations of at least 5-10 wt.% or of at least 1M, the presence of a detergent, a chelating agent and a buffer. After a tissue sample has been taken from an animal, it is immediately exposed to an aqueous composition in accordance with the present invention. The term "immediately", as used herein, is meant to refer to a period of time which ranges from 0.5 seconds to 2 minutes, preferably 0.5 seconds to 20 seconds, more preferably 0.5 seconds to 5 seconds. The subsequent exposure time during which the sample is exposed to said aqueous composition may be any period from 5 min to several hours, e. g. 1, 2, 3, ... 12, ... 24, ... 48, .. 72, ... 120, ... 168, ..., 336, ..., 720 hours. The aqueous composition in accordance with embodiments of the present invention not only assists to store and/or preserve the sample, but also to dissolve biomolecules selected from nucleic acids and proteins from such sample into the composition which can therefore be subsequently used directly without any further work-up-steps. In accordance with embodiments of the present invention, the aqueous composition comprises a buffer that is capable of buffering at a pH range from 7 to 9 at 25°C., a detergent, a salt at a concentration of at least 5-10 wt.% or of at least 1M, a chelating agent and water and, optionally, a pH-setting agent, such as an acidifying agent or an alkalizing agent. In one embodiment, the concentration of said salt is in the range of from 5-10 wt.% or from 1M-3M, preferably 1-2M, more preferably 1-1.5M. Suitable buffers are manifold. A good example is Tris.

The term "buffer", as used herein, is meant to refer to the concept of a buffer as understood by a person skilled in the art. More specifically, it refers to a combination of a weak acid and its conjugate base or of a weak base and its conjugate acid, which combination, when dissolved in water is capable of keeping the pH value in a defined range. Such "buffer" may also be referred to as a "buffer system". Suitable buffers which may be used in embodiments of the present invention include, without being limited thereto, TAPS, Bicine, Tris, Tricine, TAPSO, HEPES, TES, and MOPS.

Without wishing to be bound by any theory, the present inventors believe to have found that an aqueous composition in accordance with the present invention is capable of taking up biomolecules originating from a tissue sample of an animal, upon exposure of said tissue sample to said aqueous composition. It may be that these biomolecules are leaking out of the tissue sample due to the mechanical lysis/rupture of some cells in the tissue sample due to the tissue sampling action, such as punching, tearing or scratching, or it may be that some of the biomolecules are released from the tissue sample due to the presence of a detergent in the aqueous composition according to the present invention. The "biomolecules", that become incorporated in the aqueous composition according to the present invention, preferably dissolved therein, may be nucleic acids, proteins or both from said tissue sample. They may also or alternatively be metabolites or foreign substances, such as drugs, that were contained in the tissue sample. The "biomolecules" may be biomolecules from the animal from which the tissue sample has been taken, or they may be biomolecules stemming from a pathogen affecting the animal. In a preferred embodiment, the biomolecules that are taken up in the aqueous composition according to the present invention are nucleic acids. In another embodiment, such biomolecules are proteins. In yet a further embodiment, such biomolecules are a combination of both nucleic acids and proteins.

"Nucleic acids", as used herein, are DNA, RNA, or a mixture of both.

As a detergent of said composition, different detergents may be used. Suitable examples include SDS or N-lauroylsarcosine, preferably its sodium salt. There are also many examples for chelating agents, a suitable one being EDTA, preferably EDTA · 2 Na·2H₂O. The inventors have surprisingly found that the aqueous composition allows to keep the tissue intact as far as possible for potential future use whilst at the same time facilitating further analysis work therewith in the laboratory, as the composition dissolves biomolecules originating from the sample. These biomolecules are present in the aqueous composition at concentrations high enough to enable a further processing of such (liquid) biomolecule solution in the aqueous composition. So for example, nucleic acids and/or proteins of the animal as well as a pathogen affecting said animal, such as a virus, e.g. BVDV, may be soaked out of the tissue upon exposure of the sample to the aqueous composition. This may occur as quickly as over a period of a few minutes, i. e. when the sample is on its way during the transport from the farm to the laboratory. As used herein, the term "animal" is meant to refer to any livestock or farm animals, preferably cattle, sheep, pigs, horses and other suitable livestock animals.

The aqueous composition in accordance with embodiments of the present invention is also suitable for preserving the biomolecules dissolved therein. Hence, not only can the aqueous composition according to embodiments of the present invention serve for immediate further processing of the biomolecule(s) dissolved therein, but also for long term storage, either of the tissue sample that is being soaked therein, or of the biomolecules that have been dissolved in the aqueous composition. The inventors have surprisingly found that the biomolecules dissolved in said aqueous composition can be stored for extended periods of time therein, such as several weeks to months. Likewise, a tissue sample may be stored in the aqueous composition according to the present invention for extended periods of time, such as several weeks to months. An exemplary period of such storage of biomolecules or of a tissue sample is 1 week to 12 months, e. g. 2, 3, 4 weeks, 1, 2, 3, 4, 5, 6, 7, 8, 9, 10, 11 or 12 months.

In one embodiment, the aqueous composition does not contain proteinase at all. The inventors have surprisingly found that despite the absence of proteinase K or, even, any proteinase, it is still possible to obtain nucleic acid concentrations that are sufficiently high and pure enough for further processing, such as genotyping or the detection of pathogens, such as BVDV.

In one embodiment, the salt in the aqueous composition according to embodiments of the present invention is present at high concentrations, preferably at concentrations in the range of from 1M to 3M, preferably 1M to 2M, more preferably 1-1.5M, more preferably 1.3M to 1.5M, even more preferably around 1.4M. In one embodiment such salt is NaCl.

The inventors have surprisingly found that the use of high concentrations of salt in an aqueous composition in combination with a tissue sampling tag or tissue sampling biopsy needle allows a processing and, if desired, long-term storage/preservation of a tissue sample, without great difficulties. In particular, the aqueous composition containing high concentrations of salt in accordance with the present invention may itself be used for further analysis once a tissue sample has been exposed thereto. For many downstream processing applications, the presence of high concentrations of salt do not interfere with the further analysis of the biomolecules contained in such high salt aqueous composition. In those instances where the presence of high concentrations of salt in the aqueous composition did interfere with subsequent applications, such concentrations of high salt can easily be removed by a simple desalting step or dialysis step, without affecting the biomolecular content of the composition. For example, desalting can easily be achieved by suitable desalting columns which are commercially available, for example PD-10 desalting columns from GE Healthcare.

Many different detergents may be present in an aqueous composition according to the present invention. A preferred detergent is N-lauroylsarcosine, preferably its sodium salt. N-lauroylsarcosine appears to be particularly suitable for a high concentration of salt being present in said aqueous composition. In accordance with embodiments of the present invention, a preferred buffer is Tris and a preferred chelating agent is EDTA, preferably its disodium dihydrate.

In accordance with the present invention, the aqueous composition in accordance with the invention may be used for preserving biomolecules or tissue samples for extended periods of time, and/or for further processing of said biomolecules and/or tissue samples. Such further processing may be a diagnostic test in respect of genomic DNA of a tissue sample from an animal which may involve genotyping of such tissue sample, i. e. a detection of the presence or absence of particular genetic traits. Such genotyping may include identification of restriction fragment length polymorphisms (RFLP), random amplified polymorphic detection (RAPD) of genomic DNA, amplified fragment length polymorphism detection (AFLPD), polymerase chain reaction (PCR), DNA sequencing, allele specific oligonucleotide (ASO) probes, hybridization to nucleic acid microarrays or beads. The further processing may, in some embodiments, also or alternatively include the detection of pathogens. Such pathogens may be viruses, bacteria or eukaryotic single-celled or multicellular organisms, such as parasites. Detection of such pathogens may be via their nucleic acids or proteins thereof. Detection of nucleic acids is done as already described, using appropriate nucleic acid technology, e. g. suitable amplification/detection methodology, such as hybridization experiments, real-time PCR, hybridization on a microarray or chip or bead on which appropriate nucleic acid probes are immobilized etc. Detection of proteins may occur for example via immunosorbent assays, involving appropriate antibodies. A typical example of such an immunosorbent assay is an ELISA. Taking BVDV as an example, it is possible to detect the same, by detecting its respective nucleic acid(s) or specific proteins that are typical thereof. In one embodiment, detection of the proteins of BVDV is via any protein suitable for such purpose that appears in the BVDV, e. g. a protein selected from Npor, the capsid protein, an envelope glycoprotein, such as ERNS, E1 or E2, non-structural proteins, such as p7, NS2, NS3, NS4A, NS4B etc.

In a further embodiment, the biomolecule solution in the aqueous composition according to the present invention may also be further used for the detection of other agents, such as the presence or absence of specific markers, the presence or absence of drugs previously or supposedly administered to the animal, the presence or absence of particular hormones and/or the presence or absence of metabolites in the tissue sample and hence, in the animal.

According to one embodiment of the present invention, the use of said aqueous composition is for the detection of pathogens, in particular BVDV in a tissue sample of an animal. Such detection may be performed using any suitable means on the biomolecule solution obtained from said tissue sample and performing any suitable amplification/detection methodology on it, such as hybridization experiments, quantitative real-time PCR, hybridization on a micro array or chip or bead on which appropriate nucleic acid probes are immobilized, flow cytometry, immunohistochemistry and other suitable methodologies. Suitable tissue sampling tags or tissue sampling units/biopsy needles are commercially available from a variety of manufactures, including the present applicant Allflex Europe S.A. The aqueous composition in accordance with the present invention may be included in a suitable container, and any tissue sample obtained will be exposed to such aqueous composition in such suitable container. During use of the aqueous composition, the tissue sample is exposed to said aqueous compositioin immediately after sampling as outlined above.

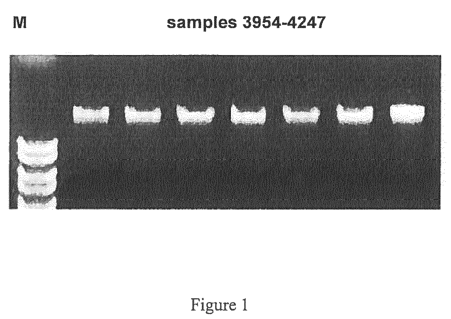

Furthermore, reference is made to the figures, wherein
Figure 1 shows the results of an exposure of tissue samples obtained from cattle to an aqueous composition in accordance with the present invention and subsequent analysis thereof in gel electrophoresis.

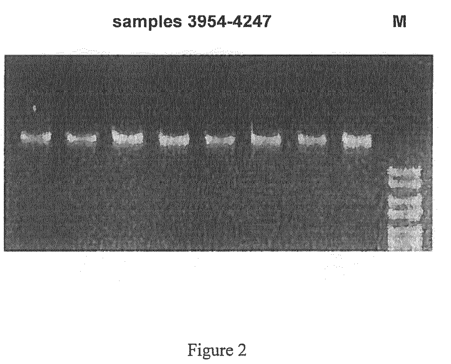

Figure 2 shows the results of an extraction of tissue samples obtained from cattle that had been kept in an aqueous composition according to the present invention for two weeks (i. e. sample archiving) wherein the samples were then re-exposed using freshly made aqueous composition in accordance with example 1 of the present invention, and the resultant solution of this re-exposure was then analyzed.

As in figure 1, the results of figure 2 are displayed as a photograph of a gel of the nucleic acids dissolved.

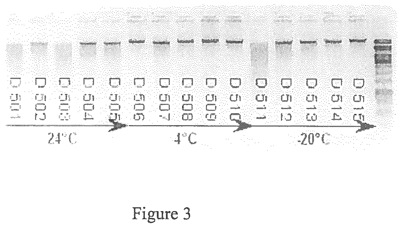

Figure 3 shows the results of a gel-electrophoretic separation of genomic DNA of samples stored for 12 months in a composition prepared in accordance with Example 1.

Furthermore, reference is made to the following examples which are given to illustrate, not to limit the present invention.

### Examples

### Example 1: Aqueous composition in accordance with embodiments of the present invention

One liter of aqueous composition in accordance with embodiments of the present invention is produced by mixing the following:
1.576 gram TRIS
0.342 gram NaOH pellets
0.744 gram EDTA.2Na.2H2O
2 gram N-Lauroylsarconsine sodium salt
81.82 gram NaCl
fill to 1 liter with ultra-pure water
in molar concentrations:
Tris MS 121.14 - 1.576g/l = 13mM
NaOH MW 39.997 - 0,342g/l = 8.55mM
EDTA 2 Na 2H₂O MW 372,24 - 0,744g/l = 1.999mM
N-Lauroylsarcosine sodium salt MW 293,38 - 2g/l = 6.81mM
NaCl MW 58.44 81,82g/l = 1.4M
make up to 11 with ultra-pure water

### Example 2: Testing of aqueous composition according to example 1 with Allflex' tissue sample tag (TST) and tissue sample unit (TSU)

Tissue sampling tags (TST) and tissue sampling units (TSU) are commercially available from the present applicant, Allflex Europe S.A.

The aim of the study in this Example is to test if Allflex' Tissue Sample Tag (TST) and Tissue Sample Unit (TSU) (= tissue sampling biopsy needle) are capable of yielding sufficient DNA for BeadChip (Illumina Inc., San Diego, CA, USA) analysis by using only the aqueous composition of example 1 the sample is preserved in, instead of the tissue sample itself.

### Material and methods

Six intact ears were received from the slaughterhouse for testing. All samples were punched several times and positive and negative controls were included to confirm test results. Both the TST and TSU were part of the study and tested independently.

Aliquots of the composition according to the present invention (Example 1) were taken from the samples after 0, 3, 19, 24, 48, 72, 168, 336, 720 hours. To test the stability of the composition, a replenishment study was performed where five weeks in a row new aqueous composition was added to 6 samples, discarding the remaining buffer in the tube.

All samples were tested on the GGP LD Bovine BeadChip (GeneSeek, Lincoln, NE, USA). Generated call rates were used to determine the quality of the data. A call rate of > 0.85 is accepted as a valid test result.

### Results

In total 156 samples were successfully genotyped. Three samples did not meet the criterion of yielding a call rate > 0.85. Samples were not retested. On average, the observed call rate, leaving out the failed samples, was 0.985.

The results from the stability study give an average call rate of 0.993 for the TSU system and 0.953 for the TST samples. The replenishment study revealed a slight drop in the call rate after replenishing the composition for the fourth time for the TSU as well as the TST samples. However, all samples still meet the inclusion criteria of a call rate of > 0.85.

### Conclusion

This study shows that using an aqueous composition in accordance with the present invention in the Allflex' sampling Tissue Sampling Technology (TSU and TST) as a source for DNA can be used in practice for successful Illumina BeadChip genotyping. Both systems, TSU as well as TST, yield comparable call rates when used for Illumina analysis.

There were no significant differences noticeable between samples analyzed after 0 - 720 hours. Directly after the ears are sampled, the aqueous composition of the present invention can be used for downstream applications. This makes the system greatly applicable for direct genetic trait and disease testing.

### Example 3: Nucleic acid extraction, archiving and genotyping trial

The aqueous composition in accordance with the present invention was assessed in terms of its suitability for dissolution and/or extraction and/or preservation of nucleic acids from tissue samples. A selection of tissue samples obtained from cattle using an Allflex tissue sampling unit (= tissue sampling biopsy needle) (TSU) were exposed to the aqueous composition in accordance with example 1 ("TSU Buffer") by placing the respective tissue sample into such composition immediately after sampling. The following results were obtained:

| Case Number | Sample | Treatment | QUBIT conc ng/pl | Nanodrop conc. ng/pl | Nanodrop A260/280 |
|---|---|---|---|---|---|
| 470003 | 3954 | TSU Buffer only | 5.09 | 22.66 | 1.87 |
| 470004 | 3970 | TSU Buffer only | 6.7 | 34 | 2.06 |
| 470005 | 3971 | TSU Buffer only | 6.81 | 32.31 | 1.92 |
| 470006 | 3995 | TSU Buffer only | 7.99 | 58.99 | 1.67 |
| 470007 | 4208 | TSU Buffer only | 6.24 | 33.93 | 1.82 |
| 470008 | 4214 | TSU Buffer only | 9.44 | 54.02 | 1.79 |
| 470009 | 4247 | TSU Buffer only | 12.4 | 53.11 | 1.85 |
| | | Average | 7.81 | 41.29 | 1.85 |

It can be seen from the table and figure 1 that sufficiently high concentrations of nucleic acid are obtained. "Qubit conc." refers to a concentration of nucleic acid as determined by using Qubit® quantitation (Thermo Fisher Scientific). The results of the exposure can also be seen in the enclosed figure 1 which clearly shows a band of (genomic) nucleic acid for the samples on a gel. (M = marker DNA). Numbers above the lanes indicate sample numbers.

Furthermore, a selection of tissue samples obtained using a tissue sampling unit (TSU) were stored in an aqueous composition according to example 1 for a period of two weeks. Thereafter the composition was exchanged and these tissues were then re-exposed to freshly made composition according to example 1. The results of such re-exposure can be summarized in the following table and figure 2:

| Case Number | Sample | Treatment | QUBIT conc ng/pl | Nanodrop conc. ng/pl | Nanodrop A260/280 |
|---|---|---|---|---|---|
| 470010 | 3954 | TSU Buffer only | | 15.59 | 1.72 |
| 470011 | 3970 | TSU Buffer only | | 15.75 | 2.25 |
| 470012 | 3971 | TSU Buffer only | | 24.72 | 2.05 |
| 470013 | 3995 | TSU Buffer only | | 94.9 | 1.47 |
| 470014 | 4208 | TSU Buffer only | | 37.57 | 1.79 |
| 470015 | 4214 | FSU Buffer only | | 45.3 | 1.59 |
| 470016 | 4247 | TSU Buffer only | | 17.27 | 1.72 |
| | | Average | | 35.87 | 1.80 |

Hence, it is clear from these data that an aqueous composition in accordance with the present invention not only is suitable to dissolve nucleic acids from a sample immediately after sampling but also to store a tissue sample therein and subsequently use such sample for further re-exposure in fresh aqueous composition of the present invention.

### Example 4: Long-term storage of tissue samples in aqueous composition according to the present invention

100 ear punch samples were collected from both ears of freshly slaughtered cattle, using an Allflex forceps system and an Allflex tissue sampling biopsy needle (TSU). Samples were stamped into plastic recipients filled with an aqueous composition according to Example 1. Such aqueous composition in accordance with the present invention is also sometimes referred to in this Example 4 as "Liquid D" or "preservative agent Liquid D" (see further below). Collection of the samples, labeling of the sample containers, and immediate shipping per courier services were carried out by Allflex. One day later, the samples reached the Laboratory in optically impeccable condition. Upon receipt, these samples were sorted as reflected by the sample labeling and information in Table 1 and were stored in darkness in the aqueous composition of Example 1.

**Table 1: Overview of sample labeling and of sample storage.**

| **Preservation** | | **Duration of storage** | | | | |
|---|---|---|---|---|---|---|
| | | **0 months** | **3 months** | **6 months** | **9 months** | **12 months** |
| Liquid D | 24°C | D101-D105 | D201-D205 | D301-D305 | D401-D405 | D501-D505 |
| | 4°C | D106-D110 | D206-D210 | D306-D310 | D406-D410 | D506-D510 |
| | -20°C | D111-D115 | D211-D215 | D311-D315 | D411-D415 | D511-D515 |

As Table 1 reflects, the first round of preparation and examination of 5 samples was processed the day after receipt of the samples (Time-Table "0-Months"), all subsequent examinations occurred after 3-months intervals (Time-Table "3, 6, 9, and 12 Months").

### Extraction of DNA from tissue samples and Determination of Quality and Quantity

All lab routines and examinations occurred according to standardized protocols.

Five samples were removed from the storage containers (24°C, 4°C, -20°C) about 30 minutes prior to extracting the DNA and equilibrated at room temperature. In general, one fraction of each ear punch sample (about 1/3) was used for DNA extraction.

Isolation of genomic DNA from the tissue samples followed a traditional protocol under moderate lysis conditions using NaOH and/or proteinase lysis. The isolated DNA was solubilized in 100µl Tris buffer (10mM).

### Quantity of Applied Sample Material

At the beginning of the project, two variants of sample quantity for lab routine were compared.

Variant 1 made use of whole ear punch samples, Variant 2 was based on a fraction of the ear punch sample (about one third of ear punch samples). This comparison appeared important, due to the imperative that processing of the samples during routine procedures has to be unfailingly continuous and reproducible. It is conceivable that the totality of the ear punch samples can be implemented at once and thus processed faster, concurrently holding DNA concentration within a narrower concentration range, thereby excluding subjective effects. As opposed to that, during routine procedure used in this example, only a fraction of the original probe was used so as to hold on to a backup.

### Photometric Assessment of Concentration and Purity of DNA

All samples were measured by Nanodrop photometrics. Each sample showed a clean spectrum of DNA with an absorption maximum at 260 nm and sparse impurity induced by protein, solvent, and salt (230 nm, 280 nm). Table 2 gives an overview of the Nanodrop values for quantity Variant 2, time table "0 months" under the two storage variety conditions.

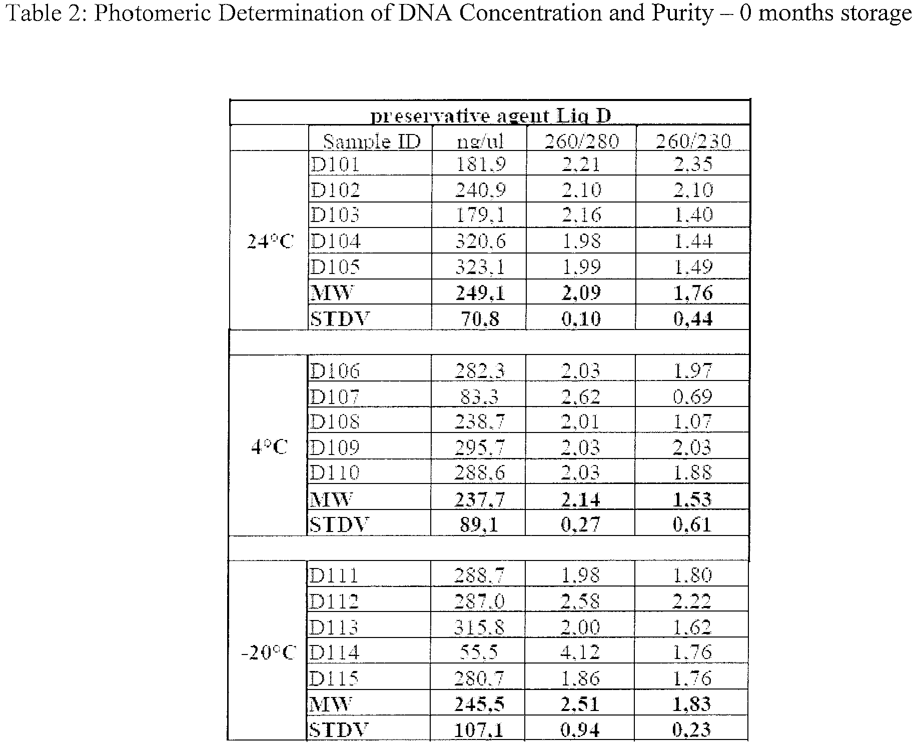

DNA concentration for Quantity Variant 1 using whole ear punch samples ranged from 191 - 829 ng/µµl (data not shown) and for Quantity Variant 2 using about 1/3 of ear punch samples ranged from 55 - 323 ng/µl. This shows significantly higher yield in DNA using all samples but also a higher spreading of measurement values. Therefore it was decided to use only a sample fraction (1/3) for all further analyses.

Tables 3 to 6 show the corresponding data after 3, 6, 9, and 12 months of storage of the tissue samples. Each sample generated a sufficient quantity of genomic DNA. Concentration of DNA showed the following ranges: 59 - 213 ng/µl (3 months), 204 - 663 ng/µl (6 months), 98 - 524 ng/µl (9 months), and 70 - 654 ng/µl (12 months) respectively.

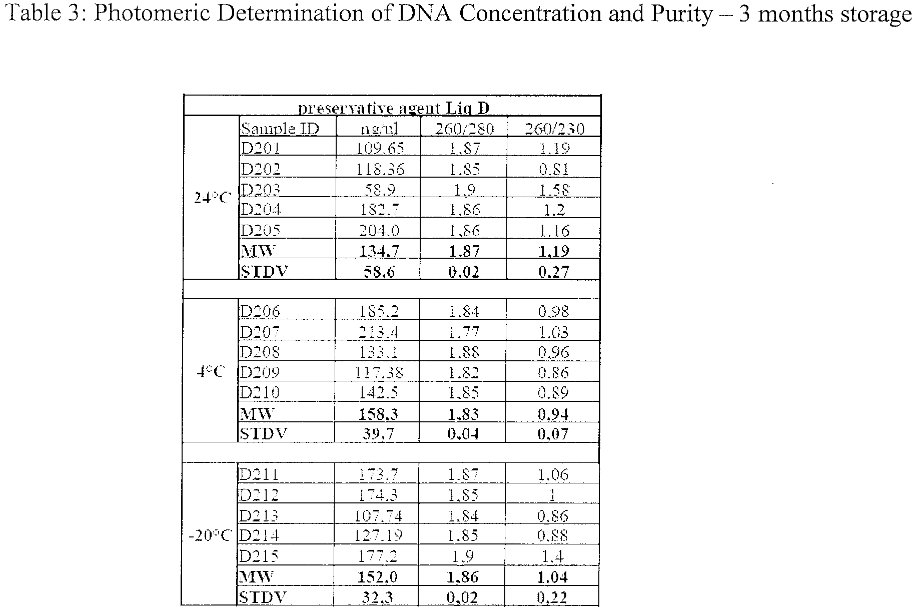

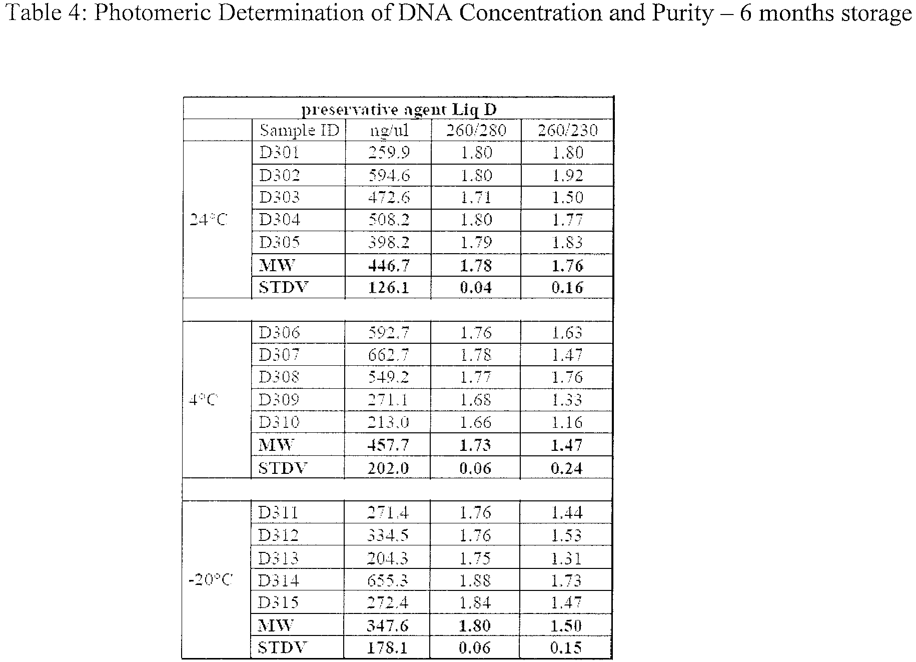

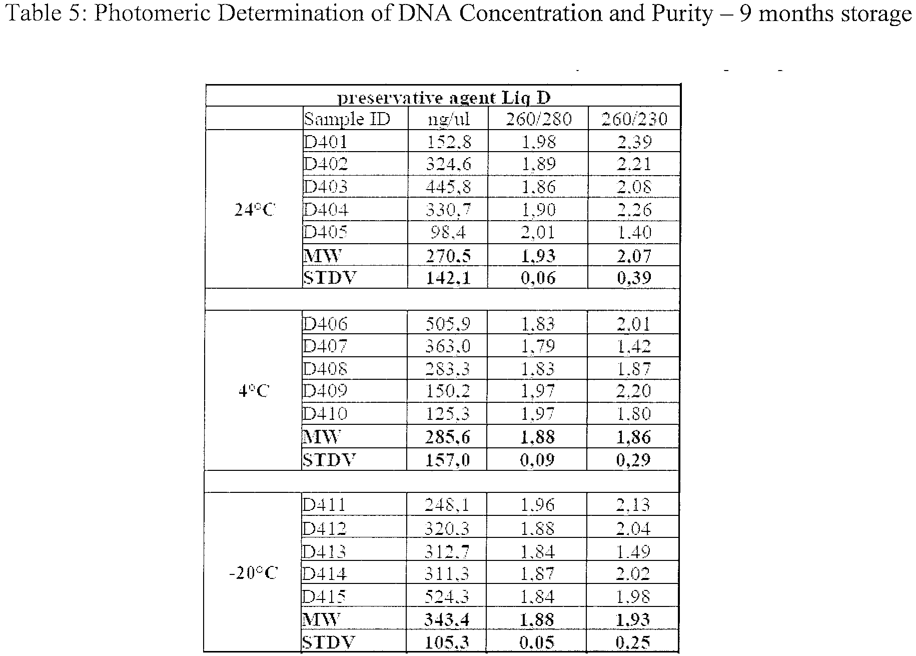

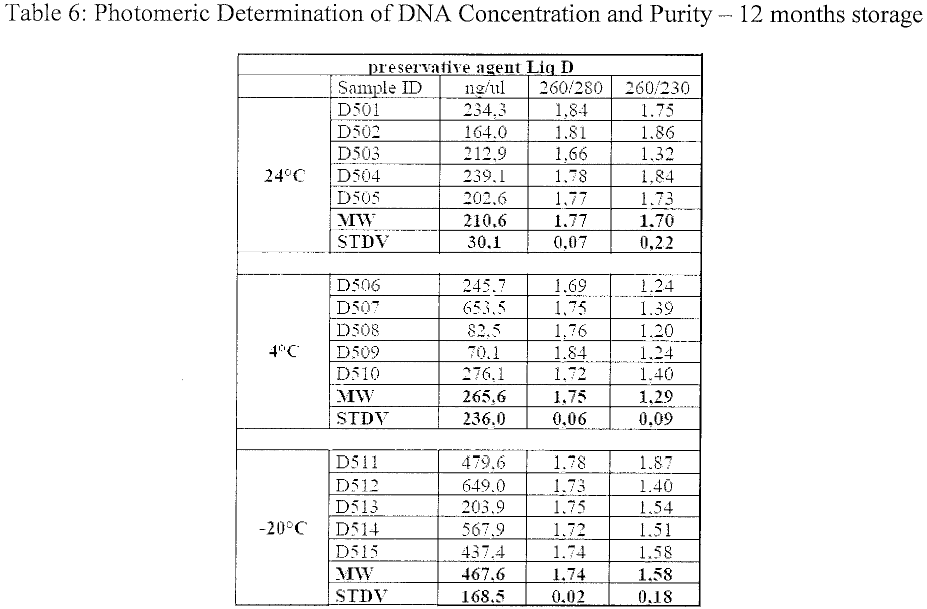

The samples obtained, after 0. 3, 6, 9 and 12 months were processed (i.e. lysed) as described above, and were gel-electrophoretically separated and analyzed. Exemplary results of such gel-electrophoretic analysis are shown in figure 3 which shows the gel-electrophoretic separation of genomic DNA for samples that had been stored for 12 months in an aqueous composition of the present invention. It becomes clear that these samples show a clearly observable high-molecular DNA band, indicating genomic DNA which leads to the conclusion that samples storage in any of the tested storage variants yielded very good DNA quality for subsequent molecular-genetic diagnostics even after a storage length of 12 months. Hence, the aqueous composition according to the present invention is also suitable for long term storage of tissue samples, which can then be used, after storage, for subsequent further processing and analysis.

## Claims

1. Use of an aqueous composition for dissolving biomolecules selected from nucleic acids, preferably DNA, and proteins, from a tissue sample of an animal and for subsequent
a) preserving said biomolecules or
b) further processing said biomolecules,
wherein said use comprises the steps:
- sampling a tissue of an animal to produce a tissue sample of said animal and
- immediately after sampling, exposing said tissue sample to an aqueous composition by contacting said sample with said composition for a defined period of time, said composition comprising
- a buffer capable of buffering at a pH range from 7 to 9 at 25°C, optionally including a pH-setting agent, such as an acidifying agent or alkalizing agent,
- a detergent
- a salt at a concentration of 5-10 wt.%
- a chelating agent, and water,
- wherein said tissue of an animal is sampled using a tissue sampling tag, preferably ear tag, or a tissue sampling biopsy needle, and wherein said sample is contacted with said composition by introducing said sample into said composition, and wherein by exposing said sample to said composition, biomolecules selected from nucleic acids and proteins, from said tissue sample become dissolved in said aqueous composition to produce a biomolecule solution,
- using said biomolecule solution for preserving said biomolecules or for further processing said biomolecules, such as detection of pathogen(s), e. g. BVDV, or genotyping, sequencing, hybridization analysis, or quantitative real-time PCR, or using said biomolecule solution for the detection of marker proteins, or marker nucleic acids, drugs, hormones or metabolites,
- optionally using said aqueous composition to store said tissue sample for later use,
wherein said aqueous composition does not contain a proteinase.

2. Use according to claim 1, wherein said salt is NaCl which is present at a concentration of 1M to 2M, preferably 1M to 1.5M, more preferably 1.3M to 1.5M, even more preferably 1.4M.

3. The use according to any of the foregoing claims, wherein said detergent in said composition is N-lauroylsarcosine, preferably N-lauroylsarcosine sodium salt.

4. The use according to any of the foregoing claims, wherein said buffer is Tris, and/or said chelating agent is EDTA, preferably EDTA disodium dihydrate, and/or said pH-setting agent is an alkali hydroxide, e.g. NaOH or KOH.

5. The use according to any of the foregoing claims, wherein said aqueous composition comprises, preferably consists of the following components:
| | |
|---|---|
| Tris | 5-20 mM, preferably 10-15 mM |
| NaOH | 5-20 mM, preferably 8-10 mM |
| N-lauroylsarcosine sodium salt | 2-15 mM, preferably 5-8 mM, more preferably 6-7 mM |
| EDTA · 2 Na·2H₂O | 1-5 mM, preferably 1-3 mM |
| NaCl | 1-3 M, preferably 1-2 M, more preferably 1-1.5 M, more preferably 1.3-1.5 M, water, |
and, optionally, a dye indicating the presence of biomolecules, preferably nucleic acids.

6. Use according to any of the foregoing claims, wherein said aqueous composition comprises, preferably consists of the following components:
| | |
|---|---|
| Tris | 13 mM |
| NaOH | 8.5-8.6 mM, preferably 8.55 mM |
| EDTA · 2 Na·2H₂O | 1.9-2.1 mM, preferably 1.99-2 mM |
| N-lauroylsarcosine sodium salt | 6-7 mM, preferably 6.7-6.9 mM, more preferably 6.8 mM |
| NaCl | 1.35-1.45 M, preferably 1.38-1.42 M, and water. |

7. Use according to any of the foregoing claims, wherein said biomolecule solution is used for further processing of said biomolecules, wherein said further processing is a detection of BVDV in said biomolecule solution and thus in said tissue sample, wherein, preferably, said detection of BVDV is done via nucleic acid amplification and detection of said amplified nucleic acids or via antibody-based detection of BVDV-proteins, such as ELISA of BVDV-proteins.

8. A system for preparing a tissue sample of an animal for subsequent
a) genotyping said tissue,
b) detection of a pathogen in said tissue, or
c) storing and preserving said tissue sample for later use,
said system comprising a tissue sampling tag, preferably tissue sampling ear tag, or a tissue sampling biopsy needle, and an aqueous composition for dissolving biomolecules from a tissue sample of an animal, said composition being contained in a container, said composition being as defined in any of claims 1-6.

## Patentansprüche

1. Verwendung einer wässrigen Zusammensetzung zum Lösen von Biomolekülen, ausgewählt aus Nukleinsäuren, bevorzugt DNA, und Proteinen, aus einer Gewebeprobe von einem Tier und zum anschließenden
a) Bewahren der Biomoleküle oder
b) weiteren Verarbeiten der Biomoleküle,
wobei die Verwendung die Schritte umfasst:
- Nehmen einer Probe eines Gewebes von einem Tier, um eine Gewebeprobe von dem Tier zu erzeugen, und
- sofort nach dem Nehmen einer Probe, Aussetzen der Gewebeprobe gegenüber einer wässrigen Zusammensetzung, indem die Probe mit der Zusammensetzung für eine definierte Zeitspanne in Kontakt gebracht wird, wobei die Zusammensetzung umfasst:
- einen Puffer, der in der Lage ist, in einem pH-Bereich von 7 bis 9 bei 25°C zu puffern, wahlweise umfassend ein pH-Einstellmittel, wie beispielsweise ein Säuerungsmittel oder Alkalisierungsmittel,
- ein Tensid,
- ein Salz in einer Konzentration von 5-10 Gew.-%,
- einen Chelatbildner und Wasser,
- wobei die Probe des Gewebes von dem Tier unter Verwendung einer Gewebeproben-Marke, bevorzugt Ohrmarke, oder einer Gewebeproben-Biopsienadel entnommen wird, und wobei die Probe mit der Zusammensetzung in Kontakt gebracht wird, indem die Probe in die Zusammensetzung eingebracht wird, und wobei durch Aussetzen der Probe gegenüber der Zusammensetzung Biomoleküle, ausgewählt aus Nukleinsäuren und Proteinen, aus der Gewebeprobe in die wässrige Zusammensetzung gelöst werden, um eine Biomolekül-Lösung zu erzeugen.
- Verwenden der Biomolekül-Lösung zum Bewahren der Biomoleküle oder zum weiteren Verarbeiten der Biomoleküle, wie beispielsweise einer Detektion von Pathogen(en), z. B. BVDV, oder Genotypisieren, Sequenzieren, Hybridisierungsanalyse oder quantitativer Echtzeit-PCR, oder Verwenden der Biomolekül-Lösung zum Detektieren von Marker-Proteinen oder Marker-Nukleinsäuren, Arzneimitteln, Hormonen oder Metaboliten,
- wahlweise Verwenden der wässrigen Zusammensetzung, um die Gewebeprobe zur späteren Verwendung zu lagern, wobei die wässrige Zusammensetzung keine Proteinase umfasst.

2. Verwendung nach Anspruch 1, wobei das Salz NaCl ist, das in einer Konzentration von 1 M bis 2 M, bevorzugt 1 M bis 1,5 M, bevorzugter 1,3 M bis 1,5 M, noch bevorzugter 1,4 M, vorliegt.

3. Verwendung nach einem der vorangehenden Ansprüche, wobei das Tensid in der Zusammensetzung N-Lauroylsarcosin ist, bevorzugt N-Lauroylsarcosin-Natriumssalz.

4. Verwendung nach einem der vorangehenden Ansprüche, wobei der Puffer Tris ist, und/oder der Chelatbildner EDTA ist, bevorzugt EDTA-Dinatriumdihydrat, und/oder das pH-Einstellmittel ein Alkalihydroxid ist, z. B. NaOH oder KOH.

5. Verwendung nach einem der vorangehenden Ansprüche, wobei die wässrige Zusammensetzung die folgenden Bestandteile umfasst, bevorzugt daraus besteht:
| | |
|---|---|
| Tris | 5-20 mM, bevorzugt 10-15 mM |
| NaOH | 5-20 mM, bevorzugt 8-10 mM |
| N-Lauroylsarcosin-Natriumssalz | 2-15 mM, bevorzugt 5-8 mM, bevorzugter 6-7 mM |
| EDTA · 2 Na·2H₂O | 1-5 mM, bevorzugt 1-3 mM |
| NaCl | 1-3 M, bevorzugt 1-2 M, bevorzugter 1-1,5 M, bevorzugter 1,3-1,5 M, |
| Wasser, | |
und wahlweise ein(en) Farbstoff, der das Vorhandensein von Biomolekülen, bevorzugt Nukleinsäuren, anzeigt.

6. Verwendung nach einem der vorangehenden Ansprüche, wobei die wässrige Zusammensetzung die folgenden Bestandteile umfasst, bevorzugt daraus besteht:
| | |
|---|---|
| Tris | 13 mM |
| NaOH | 8,5-8,6 mM, bevorzugt 8,55 mM |
| EDTA · 2 Na·2H₂O | 1,9-2,1 mM, bevorzugt 1,99-2 mM |
| N-Lauroylsarcosin-Natriumssalz | 6-7 mM, bevorzugt 6,7-6,9 mM, bevorzugter 6,8 mM |
| NaCl | 1,35-1,45M, bevorzugt 1,38-1,42 M, und Wasser. |

7. Verwendung nach einem der vorangehenden Ansprüche, wobei die Biomolekül-Lösung zum weiteren Verarbeiten der Biomoleküle verwendet wird, wobei das weitere Verarbeiten eine Detektion von BVDV in der Biomolekül-Lösung und somit in der Gewebeprobe ist, wobei, bevorzugt, die Detektion von BVDV durch Nukleinsäure-Amplifikation und Detektion der amplifizierten Nukleinsäuren oder durch Antikörperbasierte Detektion von BVDV-Proteinen, wie beispielsweise ELISA von BVDV-Proteinen, durchgeführt wird.

8. System zum Herstellen einer Gewebeprobe von einem Tier zum anschließenden
a) Genotypisieren des Gewebes,
b) Detektieren eines Pathogens in dem Gewebe oder
c) Lagern und Bewahren der Gewebeprobe zur späteren Verwendung,
wobei das System umfasst: eine Gewebeproben-Marke, bevorzugt eine Gewebeproben-Ohrmarke, oder eine Gewebeproben-Biopsienadel, und eine wässrige Zusammensetzung zum Lösen von Biomolekülen aus einer Gewebeprobe von einem Tier, wobei die Zusammensetzung in einem Behälter enthalten ist, wobei die Zusammensetzung wie in einem der Ansprüche 1-6 definiert ist.

## Revendications

1. Utilisation d'une composition aqueuse pour dissoudre des biomolécules sélectionnées parmi des acides nucléiques, de préférence de l'ADN, et des protéines, d'un échantillon de tissu d'un animal et pour ensuite
a) préserver lesdites biomolécules ou
b) réaliser un traitement supplémentaire desdites biomolécules,
où ladite utilisation comprend les étapes consistant à :
- prélever un tissu d'un animal afin de produire un échantillon de tissu dudit animal et
- immédiatement après le prélèvement, exposer ledit échantillon de tissu à une composition aqueuse par la mise en contact dudit échantillon avec ladite composition pendant une période définie, ladite composition comprenant
- un tampon capable de tamponner à une plage de pH de 7 à 9 à 25 °C, comprenant facultativement un agent d'ajustement du pH, tel qu'un agent acidifiant ou un agent alcalinisant,
- un détergent
- un sel à une concentration de 5-10 % en poids
- un agent chélateur, et de l'eau,
- où ledit tissu d'un animal est prélevé en utilisant une étiquette de prélèvement de tissu, de préférence une étiquette d'oreille, ou une aiguille de biopsie pour le prélèvement de tissu, et où ledit échantillon est mis en contact avec ladite composition en introduisant ledit échantillon dans ladite composition, et où, en exposant ledit échantillon à ladite composition, des biomolécules sélectionnées parmi des acides nucléiques et des protéines dudit échantillon de tissu sont dissoutes dans ladite composition aqueuse afin de produire une solution de biomolécules,
- utiliser ladite solution de biomolécules pour préserver lesdites biomolécules ou pour réaliser un traitement supplémentaire desdites biomolécules, tel qu'une détection d'agent(s) pathogène(s), par exemple le BVDV, ou un génotypage, un séquençage, une analyse d'hybridation, ou une PCR quantitative en temps réel, ou utiliser ladite solution de biomolécules pour détecter des protéines marqueurs, ou des acides nucléiques marqueurs, des médicaments, des hormones ou des métabolites,
- facultativement, utiliser ladite composition aqueuse pour conserver ledit échantillon de tissu pour une utilisation ultérieure, où ladite composition aqueuse ne contient pas de protéinase.

2. Utilisation selon la revendication 1, dans laquelle ledit sel est le NaCl qui est présent à une concentration de 1 M à 2 M, de préférence 1 M à 1,5 M, de manière davantage préférée 1,3 M à 1,5 M, de manière encore davantage préférée 1,4 M.

3. Utilisation selon l'une quelconque des revendications précédentes, dans laquelle ledit détergent dans ladite composition est la N-lauroylsarcosine, de préférence le sel de sodium de N-lauroylsarcosine.

4. Utilisation selon l'une quelconque des revendications précédentes, dans laquelle ledit tampon est le Tris, et/ou ledit agent chélateur est l'EDTA, de préférence l'EDTA disodique dihydraté, et/ou ledit agent d'ajustement du pH est un hydroxyde alcalin, par exemple le NaOH ou le KOH.

5. Utilisation selon l'une quelconque des revendications précédentes, dans laquelle ladite composition aqueuse comprend, de préférence consiste en les composants suivants :
| | |
|---|---|
| Tris | 5-20 mM, de préférence 10-15 mM |
| NaOH | 5-20 mM, de préférence 8-10 mM |
| Sel de sodium | 2-15 mM, de préférence 5-8 mM, de manière |
| de N-lauroylsarcosine | davantage préférée 6-7 mM |
| EDTA · 2 Na·2H₂O | 1-5 mM, de préférence 1-3 mM |
| NaCl | 1-3 M, de préférence 1-2 M, de manière |
| | davantage préférée 1-1,5 M, de manière |
| | davantage préférée 1,3-1,5 M, |
| de l'eau, | |
et, facultativement, un colorant indiquant la présence des biomolécules, de préférence des acides nucléiques.

6. Utilisation selon l'une quelconque des revendications précédentes, dans laquelle ladite composition aqueuse comprend, de préférence consiste en les composants suivants :
| | |
|---|---|
| Tris | 13 mM |
| NaOH | 8,5-8,6 mM, de préférence 8,55 mM |
| EDTA · 2 Na·2H₂O | 1,9-2,1 mM, de préférence 1,99-2 mM |
| Sel de sodium | 6-7 mM, de préférence 6,7-6,9 mM, de manière |
| de N-lauroylsarcosine | davantage préférée 6,8 mM |
| NaCl de l'eau. | 1,35-1,45 M, de préférence 1,38-1,42 M, et |
| | |

7. Utilisation selon l'une quelconque des revendications précédentes, dans laquelle ladite solution de biomolécules est utilisée pour réaliser un traitement supplémentaire desdites biomolécules, où ledit traitement supplémentaire est une détection du BVDV dans ladite solution de biomolécules et, par conséquent, dans ledit échantillon de tissu, dans laquelle, de préférence, ladite détection du BVDV est réalisée via une amplification d'acide nucléique et une détection desdits acides nucléiques amplifiés ou via une détection à base d'anticorps des protéines du BVDV, tel qu'un ELISA des protéines du BVDV.

8. Système pour préparer un échantillon de tissu d'un animal pour ensuite
a) réaliser un génotypage dudit tissu,
b) détecter un agent pathogène dans ledit tissu, ou
c) conserver et préserver ledit échantillon de tissu pour une utilisation ultérieure,
ledit système comprenant une étiquette de prélèvement de tissu, de préférence une étiquette d'oreille de prélèvement de tissu, ou une aiguille de biopsie pour le prélèvement de tissu, et une composition aqueuse pour dissoudre des biomolécules d'un échantillon de tissu d'un animal, ladite composition étant contenue dans un récipient, ladite composition étant telle que définie dans l'une quelconque des revendications 1 à 6.
